# EUROPEAN PATENT APPLICATION

(11) **EP 3 607 932 A1**
(43) Date of publication of application: **12.02.2020**
(21) Application number: 18781130.2
(22) Date of filing: 03.04.2018
(51) Int. Cl.: A61K 8/02, A61K 8/19, A61Q 19/00

(54) **DROPLET-RELEASING COSMETIC MATERIAL**

(30) Priority: 03.04.2017 KR 20170042863
(71) Applicant: Sunjin Beauty Science Co., Ltd., Ansan-si, Gyeonggi-do 15612 (KR)
(72) Inventor: LEE, Sung Ho, Ansan-si Gyeonggi-do 15612 (KR)
(74) Representative: Bockhorni & Brüntjen Partnerschaft Patentanwälte mbB
(86) International application number: PCT/KR2018/003943
(87) International publication number: WO 2018/186669

(57) **Abstract**

The present invention relates to a droplet-releasing cosmetics material, in particular cosmetics material comprising a polyhedral complex containing: in cosmetics which release a droplet by rubbing, a) a polyhedral structure wherein an interior is composed with a plurality of plate-like materials and has a space therein; and b) droplets contained in a space inside the polyhedral structure.

A cosmetics material according to the present invention releases a droplet by rubbing in use thereby having a visual effect and excellent feeling in sue and is silicone polymer-free type capable of solving the problem of side effects by the silicone polymer and the user's discomfort and has benefit of excellent stability in the cosmetic material of the polyhedral complex.

## Description

### [Technical Field]

The present invention relates to a droplet-releasing cosmetics material and cosmetic, respectively, and in particular, to a droplet-releasing cosmetic in which the droplet is released by rubbing in use thereby having a visual effect and excellent feeling and which is silicone polymer-free type capable of solving the problem of side effects by the silicone polymer and the user's discomfort and having excellent stability in the cosmetic of the polyhedral complex.

### [Background Art]

The contents described below merely provide background information related to the present invention and do not constitute a prior art.

Recently, droplet-releasing cosmetics such as water drop cosmetics have been preferred for cosmetics. Moisture release cosmetics bursts water by rubbing when in use, have a unique visual effect to the user, and can have a good feeling such as moist and cool feeling.

The structural component that releases moisture in the moisture-releasing cosmetics is highly stable in the cosmetics and because the moisture should be well released when rubbing, the conventional moisture-releasing cosmetics mostly used silicone polymer as a structure of the component for releasing moisture.

However, silicone components used in cosmetics have been reported to have various harmful effects on human body such as CMR (Carcinogenic, Mutagenic, Reprotoxic), and thus the use of cosmetics has limited starting from Europe.

Accordingly, there is an urgent need for the development of cosmetics having a moisture release effect without a silicone component.

### [Disclosure]

### [Technical Problem]

It is an object of the present invention to provide a droplet-releasing cosmetics material in which the droplet is released by rubbing in use thereby having a visual effect and excellent feeling and which is silicone polymer-free type capable of solving the problem of side effects by the silicone polymer and the user's discomfort and having excellent stability in the cosmetic material of the polyhedral complex.

### [Technical Solution]

To solve the above problems, the present invention provides cosmetics material comprising a polyhedral complex containing: in cosmetics which release a droplet by rubbing, a) a polyhedral structure wherein an interior is composed with a plurality of plate-like materials and has a space therein; and b) droplets contained in a space inside the polyhedral structure.

The plate-like materials of the polyhedral structure may have a lipophilic property in faces thereof and has a hydroxyl group in corners thereof.

The polyhedral structure may be a thixotropic polyhedron.

The polyhedral structure may be pentahedron (5-) to pentactahedron (500-).

The surfaces of the plate-like material may be treated with a quaternary ammonium salt having a lipophilic residue.

The droplet may further comprise an emulsifier.

The droplet may further comprise oil.

The polyhedral complex may have a size of 5 to 60 um in diameter.

The polyhedral structure and the droplets of the polyhedral complex may contain 0.1-20 parts by weight: 80-99.9 parts by weight.

The content of the droplets may be 10 to 97% by weight.

### [Advantageous Effects]

A droplet-releasing cosmetics material according to the present invention releases a droplet by rubbing in use thereby having a visual effect and excellent feeling and is silicone polymer-free type capable of solving the problem of side effects by the silicone polymer and the user's discomfort and has benefit of excellent stability in the cosmetic material of the polyhedral complex.

### [Description of Drawings]

FIG. 1 is a schematic diagram showing a state in which the moisture of the moisture-releasing cosmetics is released.
FIG. 2 shows a two-dimensional schematic diagram of a polyhedral complex according to an example of the present invention.
FIG. 3 shows a three-dimensional schematic diagram of a polyhedral complex according to an example of the present invention.
FIG. 4 is a schematic diagram illustrating the principle that the adjacent plate-like material forming the polyhedral complex of the present invention forms a card polyhedral structure.
FIG. 5 is an electron micrograph of droplet-releasing cosmetics material according to an example of the present invention.
FIG. 6 is a photograph when the cosmetics material according to an example of the present invention is coated by rubbing on skin.

### [Best Mode]

Hereinafter, examples of the present invention will be described in detail with reference to the accompanying drawings so that those skilled in the art may easily implement the present invention. However, the invention may be embodied in many different forms and it is not limited to the example explained herein. In the drawings, parts irrelevant to the description are omitted in order to clearly describe the present invention, and similar reference numerals are designated for similar parts throughout the specification.

Throughout the specification, when a part "includes" a component, it means that it can further include other components, not to exclude other components unless otherwise stated.

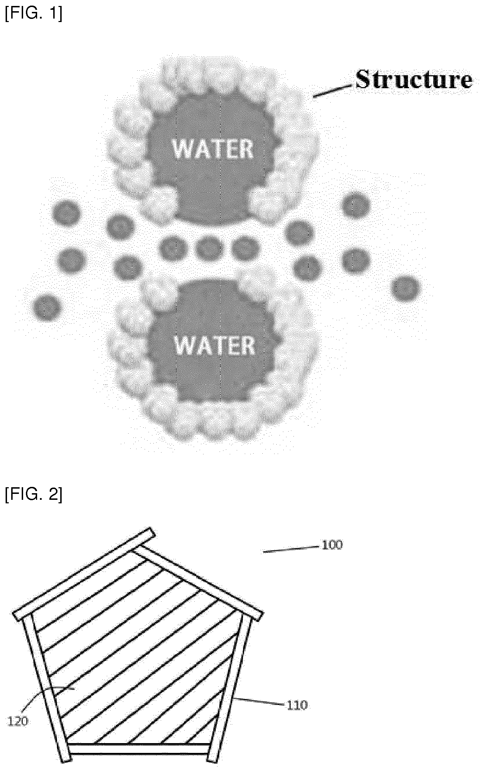

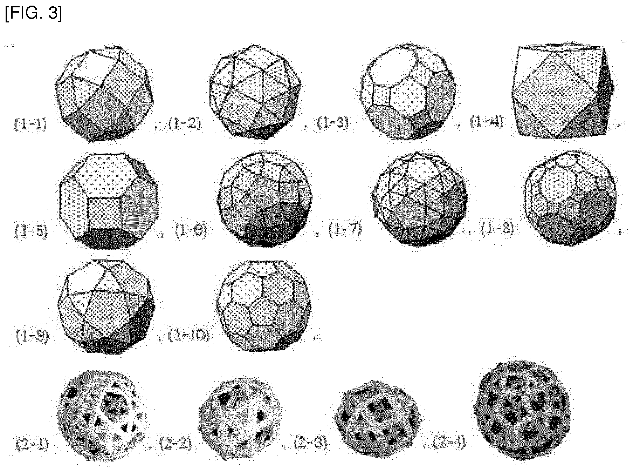

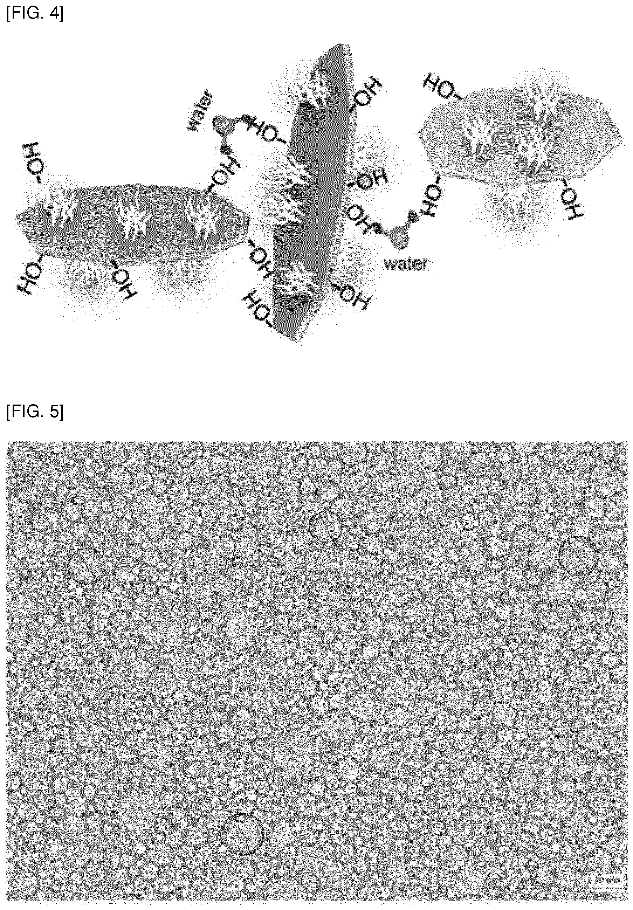

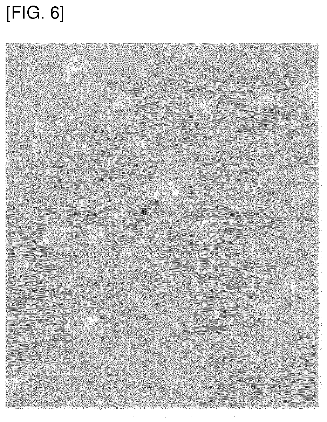

FIG. 1 is a schematic diagram showing a state in which the moisture of the moisture-releasing cosmetics is released; FIG. 2 shows a two-dimensional schematic diagram of a polyhedral complex according to an example of the present invention; FIG. 3 shows a three-dimensional schematic diagram of a polyhedral complex according to an example of the present invention; FIG. 4 is a schematic diagram illustrating the principle that the adjacent plate-like material forming the polyhedral complex of the present invention forms a polyhedral structure; FIG. 5 is an electron micrograph of droplet-releasing cosmetics material according to an example of the present invention; and FIG. 6 is a photograph when the cosmetics material according to an example of the present invention is coated by rubbing on skin.

Droplet (specifically, water) releasing cosmetics may have a form in which the structure receives moisture inside thereof as shown in FIG. 1 and in use, the structure may be deformed by rubbing to a form of releasing water.

The cosmetics material of the present invention comprises a polyhedral complex 100 containing: in cosmetics which release a droplet by rubbing, a) a polyhedral structure 110 wherein an interior is composed with a plurality of plate-like materials and has a space therein; and b) droplets 120 contained in a space inside the polyhedral structure.

The polyhedral complex 100 may be formed through thixotropic bonding in edge portion between adjacent plate materials while surrounded by the plurality of plate materials as shown in FIG. 4. Even after the bond is unbound by the impact, the bond is formed again to maintain the polyhedral complex 100.

The plate-like material may have a lipophilic property in faces thereof and a hydroxyl group in corners thereof. Specifically, the plate-like material may be used after swelling a smectite clay mineral to separate into individual plate-like material and then changing the faces with a lipophilic material. Individually separated plate-like materials may be purchased commercially, for example, Elementis Co.

The smectite clay mineral is not particularly limited, and specifically, may be hectorite or bentonite, and preferably hectorite. In this case, it is possible to form a polyhedral complex 100 having more superior stability.

The lipophilic material is not particularly limited as long as it can impart lipophilic property to the faces of the plate-like material, specifically, may be a material having an aliphatic alkyl moiety of 5 to 22 carbon atoms. More specifically, the material having an aliphatic alkyl moiety of 5 to 22 carbon atoms may be a quaternary ammonium salt.

The plate-like material may be Disteardimonium Hectorite.

The plate-like material is not particularly limited in shape of the faces, may be a circular, triangle to decagon, or an amorphous material and two or more kinds may be mixed.

In addition, the plate-like material can be selected by optionally adjusting the thickness, the length of the long axis and the short axis. Specifically, the thickness may be 0.0005-0.005 um, the long axis may be 0.5-1.5 um. Within the above range, it is particularly suitable for forming the polyhedral complex 100 having excellent thixotropy and dispersibility.

The plate-like material may be a mixture of two or more different materials. For example, a mixture of a material having a length of 0.8 um and a material having a length of 1.2 um may be used. In this case, it is suitable for forming a polyhedral complex 100 having excellent shape stability.

In addition, the droplet 120 in the cosmetics material of the present invention is not particularly limited as long as it is a liquid, and specially, it may be an aqueous phase (water), but is not limited thereto. The aqueous phase may be water in which known components capable of being used in cosmetics is dissolved. Specific examples of the known components may include ions, flavors, extracts, nutrients or other functional ingredients used in cosmetics in the amount of conventional additives used (0.001 to 10% by weight) and mixture of at least two known components can be used.

In the present invention, the content of the polyhedral structure 110 and the droplet 120 of the polyhedral complex 100 may be optionally adjusted, and may be contained in a ratio of 0.1-20 parts by weight: 80 to 99.9 parts by weight. In this case, the polyhedral complex 100 having excellent dispersibility and stability can be formed.

The droplet 120 may further comprise an emulsifier as needed. In this case, a polyhedral complex having better shape stability may be formed. The emulsifier is not particularly limited as long as it can be used in cosmetics, the content can be optionally adjusted and specifically, the polyhedral structure and emulsifier may be included in a ratio of 0.1-20 parts by weight: 1 to 50 parts by weight. Specific examples of the emulsifier may be at least one emulsifiers selected from the group consisting of Polyglyceryl-4 Isostearate(HLB 5), Polyglyceryl-3 Polyricinoleate(HLB 4), Polyglyceryl-3 Diisostearate(HLB 5.5), Polyglyceryl-4 Oleate(HLB 3), Polyglyceryl-4 Diisostearate/Polyhydroxystearate/Sebacate(HLB 5), Diisostearoyl Polyglyceryl-3 Dimer Dilinoleate(HLB 5), Polyglyceryl - 3 Polyricinoleate (and) Sorbitan Isostearate(HLB 4.2), Glyceryl Laurate(HLB 5.2), Sorbitan Oleate (HLB 4.3) and Sorbitan Sesquioleate(HLB 3.7).

The droplet 120 may further comprise oil as needed. In this case, the polyhedral complex 100 having excellent dispersibility and shape stability can be formed, and the feeling in use of the cosmetic can be improved.

The oil is not particularly limited as long as it can be used in cosmetics, and the content can be optionally adjusted, and specifically, the polyhedral structure and the oil may include 0.1 to 20 parts by weight: 1 to 50 parts by weight. Specific examples of the oil may be at least one oil selected from the group consisting of Coco-Caprylate/Caprate, Caprylic/Capric Triglyceride, Dicaprylyl Carbonate, Myristyl Myristate, Dicaprylyl Ether, Cetyl Palmitate, Octyldodecanol, Hexyldecanol, Hexyldecyl Stearate, Cetyl Palmitate, Isostearyl Isostearate, Decyl Oleate, Hydrogenated Vegetable Oil, Methylheptyl Isostearate, Nigella Sativa Seed Oil, Camellia Oleifera Seed Oil and Passiflora Incarnata Oil.

In the present invention, the polyhedral complex 100 may be pentahedron (5-) to pentactahedron (500-) and two-dimensionally polyhedron as shown in FIG. 2, three-dimensionally one of the polyhedron as shown in FIG. 3. Within the above range, the droplet 120 is easily released by rubbing when the cosmetic is used and the stability of the polyhedral complex 100 in the cosmetics material is excellent. In addition, the polyhedral complex 100 may have a structure in which pores are blocked as shown in 1-1 to 1-10 of FIG. 3, or may have a structure having pores as in 2-1 to 2-4. It may have smooth edges, or may have a structure in which a part of the plate-like material protrudes. If there is a pore, the cross-sectional size of the largest pore is preferably within 50% of the maximum cross-sectional size of the droplet 120, preferably, the cross-sectional size of the largest pore is within 10% of the maximum cross-sectional size of the droplet 120. In this case, since the thixotropic properties of the polyhedral complex 100 are excellent, the cosmetics material has more excellent stability.

The size of the polyhedral complex 100 in the cosmetics material of the present invention may be 5 to 60 um in diameter, preferably 10 to 45 um, and more preferably at least 50% of 10 to 45 um in diameter. In this case, it is easy to release the droplets 120 by rubbing when use of cosmetics material, and the stability of the polyhedral complex 100 in the cosmetics is more excellent. If the size of the polyhedral complex 100 is too small, it may not be easy to release the droplet 120 during the use, and if the size of the polyhedral complex 100 is too large, the stability in cosmetics material may be significantly reduced.

A droplet-releasing cosmetics material according to the present invention can be embodied in various formulations capable of releasing the droplet 120, including the known components, the content of the droplet can be optionally adjusted, specific examples of the content of the droplet 120 is 10 to 97 weight%, preferably at least 30 weight% by weight of water, and more preferably at least 50 weight% of water. Within the above range, it is easy to release the droplets 120 by rubbing when using the cosmetics material, and the stability of the polyhedral complex 100 in the cosmetics material is more excellent, and it can have an excellent feeling such as moist and cool feeling to the users.

Droplet release cosmetics material according to the present invention may be prepared by mixing the material constituting the polyhedral structure 110 to the cosmetics material comprising a droplet 120. As a specific example, the cream may be prepared by mixing 1 to 20 parts by weight of the plate-like material constituting the polyhedral structure 110 to 80 to 97 parts by weight of the cream cosmetics material including 50 to 90 parts by weight of aqueous droplets. It may further include 1 to 30 parts by weight of known components that can be used in cosmetics, such as moisturizers, softeners, as needed, but it is not limited thereto. In addition, the droplets may further comprise 1 to 50 parts by weight of an emulsifier. In addition, the droplet may further comprise 1 to 50 parts by weight of oil.

As specific example, a cream was prepared by mixing 5 weight% of mixture of 10 parts by weight of Disteardimonium Hectorite, 40 parts by weight of POLYGLYCERYL-4 ISOSTEARATE and 50 parts by weight of Coco-Caprylate/Caprate, 7 weight% of a moisturizer, 0.5 weight% of NaCl, 3 weight% of glycerin, 1.7 weight% of propanediol, 0.5 weight% of plant extract, 0.7 weight% of hexanediol, 0.03 weight% of fragrance and the reminder of water, An electron micrograph of the cream is shown in FIG. 5 and a picture of the cream when rubbed on the skin is shown in FIG. 6.

As shown in FIG. 5, it can be confirmed that the droplet 120 is stably received in the polyhedral structure 110 in the cosmetics material of the present invention, and as shown in FIG. 5, the effect of releasing the droplet 120 is excellent.

A cosmetics material releasing a droplet 120 of the present invention can release the droplet 120 by rubbing in use thereby having a visual effect and excellent feeling in use and is silicone polymer-free type capable of solving the problem of side effects by the silicone polymer and the user's discomfort and has excellent stability in the cosmetic material of the polyhedral complex 110.

Simple modifications and changes of the present invention can be readily performed by those skilled in the art, and all such modifications and changes are included within the scope of the present invention.

## Claims

1. Cosmetic for releasing droplets by rubbing, the cosmetic comprising a polyhedral complex containing:
a) a polyhedral structure wherein an interior is composed with a plurality of plate-like materials and has a space therein; and
b) droplets contained in the space inside the polyhedral structure.

2. The cosmetic of claim 1, wherein the plate-like material of the polyhedral structure has a lipophilic property in faces thereof and has a hydroxyl group in corners thereof.

3. The cosmetic of claim 1, wherein the polyhedral structure is a thixotropic polyhedron.

4. The cosmetic of claim 1, wherein the polyhedral structure is pentahedron (5-) to pentactahedron (500-)

5. The cosmetic of claim 1, wherein surfaces of the plate-like material are treated with a quaternary ammonium salt having a lipophilic residue.

6. The cosmetic of claim 1, wherein the droplet further comprises an emulsifier.

7. The cosmetic of claim 1, wherein the droplet further comprises oil.

8. The cosmetic of claim 1, wherein the polyhedral complex has a size of 5 to 60 *µ*m in diameter.

9. The cosmetic of claim 1, wherein the polyhedral structure and the droplets of the polyhedral complex contain 0.1-20 parts by weight: 80-99.9 parts by weight.

10. The cosmetic of claim 1, wherein content of the droplets is 10 to 97% by weight.
